(19) Europäisches Patentamt
European Patent Office
Office européen des brevets

(11) Veröffentlichungsnummer : **0 111 160**
**B1**

(12) **EUROPÄISCHE PATENTSCHRIFT**

(45) Veröffentlichungstag der Patentschrift : **22.10.86**

(21) Anmeldenummer : **83111080.4**

(22) Anmeldetag : **07.11.83**

(51) Int. Cl.⁴ : **C 07 D241/18**, C 07 D241/24, C 07 D241/20

(54) **Verfahren zur Herstellung von 2-Halogen- und 2-Cyanpyrazinen.**

(30) Priorität : **15.11.82 DE 3242266**

(43) Veröffentlichungstag der Anmeldung : **20.06.84 Patentblatt 84/25**

(45) Bekanntmachung des Hinweises auf die Patenterteilung : **22.10.86 Patentblatt 86/43**

(84) Benannte Vertragsstaaten : **BE CH DE FR GB IT LI**

(56) Entgegenhaltungen :
**US-A- 4 254 125**
**CHEMICAL ABSTRACTS, Band 90, 1979, Seite 583, Nr. 6352w, Columbus, Ohio, USA H. FOKS et al.: "Pyrazine derivatives. VI. Synthesis and tuberculostatic activity of 6-alkoxy- and 6-phenoxipyrazine-2-carbothioamides"**
**JOURNAL OF THE CHEMICAL SOCIETY (C), 1971 (18), Seiten 2973-2976, London, GB. G.W.H. CHEESEMAN et al.: "Pyrazines. Part III. Some nucleophilic substitution reactions of chloropyrazines"**
**JOURNAL OF THE CHEMICAL SOCIETY (C), 1971 (18), Seiten 2977-2979, London, GB. G.W.H. CHEESEMAN et al.: "Pyrazines. Part IV. 2,6-Dihydroxy-3,5,diphenylpyrazine and related compounds"**

(73) Patentinhaber : **BASF Aktiengesellschaft**
**Carl-Bosch-Strasse 38**
**D-6700 Ludwigshafen (DE)**

(72) Erfinder : **Kroener, Michael, Dr.**
**Eislebener Weg 8**
**D-6800 Mannheim 42 (DE)**
Erfinder : **Schenk, Walter, Dr.**
**Holzweg 28**
**D-6702 Bad Duerkheim (DE)**

**EP 0 111 160 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein Verfahren zur Herstellung von 2-Halogen- und 2-Cyanpyrazinen durch Umsetzung von 2-Aminopyrazinen bei einer Temperatur von —50° bis + 50 °C a) mit Alkalinitriten oder Alkylnitriten in Gegenwart von Wasser und/oder organischen Lösungsmitteln, a1) mit Tetrafluorborwasserstoffsäure oder a2) Halogenwasserstoffsäuren in Form einer 10- bis 80-gewichtsprozentigen Lösung und in einer Menge von 1 bis 5 Mol Halogenwasserstoffsäure je Mol 2-Aminopyrazin oder b) mit Nitrosylhalogeniden in Gegenwart von unter den Reaktionsbedingungen inerten organischen Lösungsmitteln und gewünschtenfalls anschließende Umsetzung der so gebildeten 2-Halogenpyrazine mit Kupfercyanid und gegebenenfalls Alkali- und/oder Erdalkalicyaniden bei 80 bis 200 °C.

Es ist bekannt, 2-bromsubstituierte Pyrazine aus Hydroxipyrazinen durch Umsetzung mit Phosphorbromiden (JACS *78*, (1956) 2141-2144) herzustellen. Die Reaktion liefert in einigen Fällen der Herstellung substituierter Pyrazine mäßige Ausbeuten und erfordert hohe Phosphorhalogenidüberschüsse. Die Umsetzung wird bei 50 bis 145 °C durchgeführt. Als Substituenten in 3- und 5-Stellung werden die Methyl, Ethyl, Propyl, Isopropyl, und Phenylgruppe gezeigt. Ebenfalls wird die Umsetzung solcher 2-Bromverbindungen mit Kupfercyanid in γ-Picolin oder Pyridin beschrieben. Für fast alle Umsetzungen muß ein Verhältnis 0,168 Mol Kupfercyanid zu 0,05 Mol 2-Brompyrazin eingehalten werden, wobei man Ausbeuten von 75 bis 82 % der Theorie an mit 1 oder 2 Alkylgruppen substituierten 2-Cyanpyrazinen erhält.

Eine vor allem technisch interessante Synthese (J. Org. Chem. *26*, 2356-3360 (1961) bedient sich der Halogenierung von Alkylpyrazinen, bei der jedoch schwer trennbare Isomerengemische entstehen

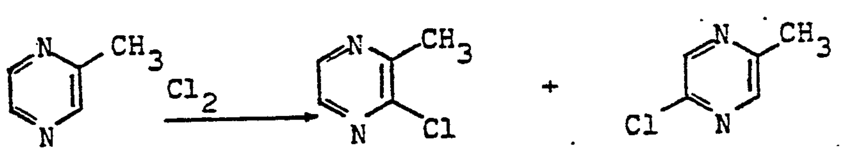


Eine Synthese von Halogenpyrazinen aus Aminopyrazinen mit Hilfe der Sandmeyerreaktion ist bisher nicht gelungen (Advances in Heterocyclic Chemistry *14*, (1972), 166). Die intermediär auftretenden Diazoniumsalze zersetzen sich bei der in JACS *68*, 400-402 (1946) beschriebenen Arbeitsweise mit Schwefelsäure und Natriumnitrit zu Pyrazinonen. Man erhält ebenfalls Pyrazinone bei der in Journal Chem. Soc. 1947, 370-372 beschriebenen Arbeitsweise mit n-Chlorwasserstoffsäure und Natriumnitrit während 5 Minuten bei 0 °C, bei Raumtemperatur während 120 Minuten und 5 Minuten bei 60 °C. Die Ausbeuten an unsubstituiertem Pyrazinon bei den beiden Verfahren betragen 65 bzw. 30 %. Jour. Amer. Chem. Soc. (loc. cit. Seite 400) gibt außerdem an, daß Pyrazin im Gegensatz zu Pyrimidin sich nicht leicht bei den üblichen Substituierungsreaktionen, z. B. Halogenierung, umsetzt. Weiterhin wird als beste Herstellungsmethode von 2-Halogenpyrazinen angesehen (loc. cit. Seite 401), daß man zuerst 2-Aminopyrazine über die Diazotierung und Hydrolyse zu 2-Hydroxypyrazinen und diese dann mit einem Phosphorhalogenid zu den entsprechenden Halogenpyrazinen umsetzt ; man erhält z. B. im Falle des 2-Hydroxypyrazins eine Ausbeute von 58 % an Chlorpyrazin. Bei Verwendung von Phosphorbromiden liefert die Arbeitsweise Gemische von Mono- und Dibrompyrazinen.

Weitere Darstellungsmethoden für Pyrazinderivate sind in US-PS 4 254 125, Chemical Abstracts, Bd. 90, 1979, Seite 583, Nr. 6352w und in Journal of the Chemical Society (C), 1971 (18), Seiten 2973-2976 u. 2977-2979 beschrieben.

Es wurde nun gefunden, daß man 2-Halogen- oder 2-Cyanpyrazine der Formel I

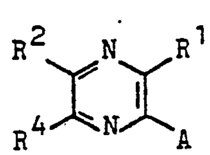

(I)

worin A eine Halogenatom oder eine Cyangruppe bedeutet, $R^1$ und $R^2$ gleich oder verschieden sein können und jeweils für ein Wasserstoffatom, einen aliphatischen, cycloaliphatischen, araliphatischen oder aromatischen Rest stehen, $R^4$ ein Wasserstoffatom oder den Rest $R^3Y$— bedeutet, worin $R^3$ für einen aliphatischen, cycloaliphatischen, araliphatischen oder aromatischen Rest steht und Y für ein Sauerstoffatom oder ein Schwefelatom steht, durch Umsetzung von 2-substituierten Pyrazinen mit Halogenverbindungen zu 2-Halogenpyrazinen und gewünschtenfalls Umsetzung von 2-Halogenpyrazinen mit Kupfercyanid in Gegenwart von unter den Reaktionsbedingungen inerten heterocyclischen Lösungsmitteln, vorteilhaft erhält, wenn man 2-Aminopyrazine der Formel II

(Siehe Schema Seite 3 f.)

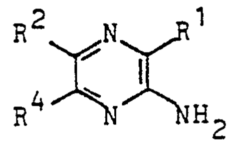

(II)

worin $R^1$, $R^2$ und $R^4$ die vorgenannte Bedeutung besitzen, bei einer Temperatur von —50 bis + 50 °C

a) mit Alkalinitriten oder Alkylnitriten in Gegenwart von Wasser und/oder organischen Lösungsmitteln a1) mit Tetrafluorborsäure oder a2) Halogenwasserstoffsäuren der Formel

$$H{-}X \qquad \text{(III)}$$

worin X ein Halogenatom bedeutet, in Form einer 10- bis 80-gewichtsprozentigen Lösung und mit Ausgangsstoff III in einer Menge von 1 bis 5 Mol Ausgangsstoff III je Mol Ausgangsstoff II, oder

b) mit Nitrosylhalogeniden in Gegenwart von unter den Reaktionsbedingungen inerten organischen Lösungsmitteln

umsetzt und gewünschtenfalls die so erhaltenen 2-Halogenpyrazine der Formel

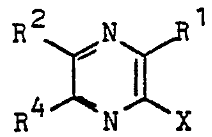

(IV)

worin $R^1$, $R^2$, $R^4$ und X die vorgenannte Bedeutung besitzen mit Kupfercyanid und gegebenenfalls Alkali- und/oder Erdalkalicyaniden bei einer Temperatur von 80 bis 200 °C umsetzt.

Weiterhin wurden die neuen 2-Halogen- und 2-Cyanpyrazine der Formel I

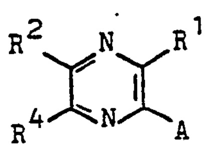

(I)

worin A ein Halogenatom oder eine Cyangruppe bedeutet, $R^1$, $R^2$ und $R^3$ gleich oder verschieden sein können und jeweils für einen aliphatischen, cycloaliphatischen, araliphatischen oder aromatischen Rest stehen, $R^1$ und $R^2$ auch jeweils ein Wasserstoffatom bedeuten können, Y für ein Sauerstoffatom oder ein Schwefelatom steht, $R^4$ den Rest $R^3Y$ bedeutet, worin $R^3$ und Y die vorgenannte Bedeutung besitzen, wobei, wenn A eine Cyangruppe bedeutet, $R^4$ auch ein Wasserstoffatom sein kann, sofern $R^1$ und/oder $R^2$ kein Wasserstoffatom, keinen Alkylrest mit 1 bis 3 Kohlenstoffatomen und keine Phenylgruppe bezeichnen, gefunden.

Die Umsetzung kann für den Fall der Verwendung von 3,5-Dimethyl-2-aminopyrazin, Salzsäure, Natriumnitrit, bzw. Nitrosylbromid bzw. Kupfercyanid durch die folgenden Formeln wiedergegeben werden :

a)

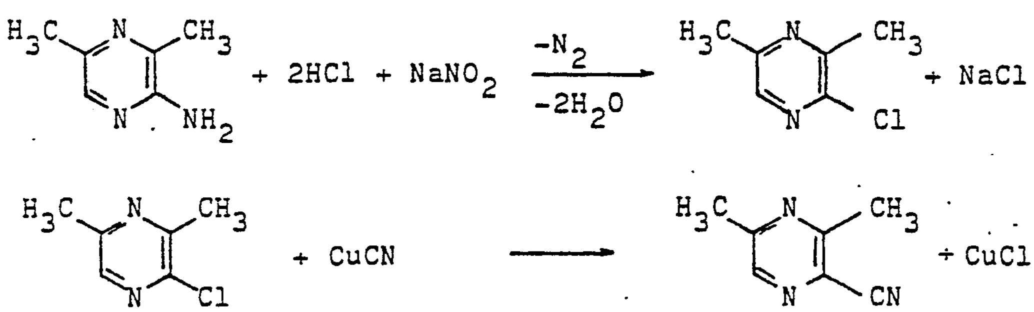


b)

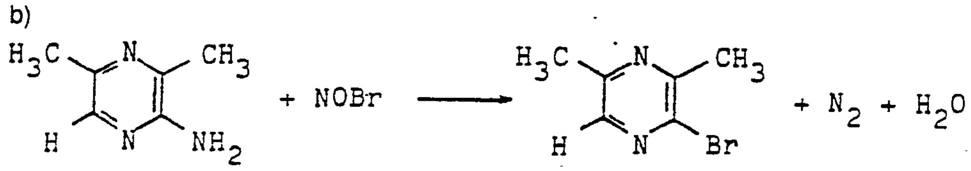


Im Vergleich zu den beschriebenen Verfahren liefert das Verfahren nach der Erfindung auf

einfacherem und wirtschaftlicherem Wege 2-Halogen- und 2-Cyanpyrazine in guter Ausbeute und Reinheit und besserer Raum-Zeit-Ausbeute. Eine mehrstufige Herstellungsweise wird vermieden. Alle diese vorteilhaften Eigenschaften sind im Hinblick auf den Stand der Technik überraschend. Man hätte im Hinblick auf den Stand der Technik erwartet, daß 2-Hydroxypyrazine zumindest als Hauptprodukt der Umsetzung entstehen und 2-Halogenpyrazine, wenn überhaupt, dann nur in unwesentlichen Mengen, auftreten. Weiterhin konnte im Hinblick auf die bekannte 2-Cyanpyrazinherstellung nicht erwartet werden, daß Kupfercyanid durch Alkali- oder Erdalkalicyanide und noch dazu in überwiegender Menge ersetzt werden kann. Überraschend erhält man in Hinblick auf die Lehre von Jour. Amer. Chem. Soc. (loc. cit.) mit wesentlich geringeren Überschüssen von Kupfercyanid höhere Ausbeuten an 2-Cyanpyrazinen I.

Bevorzugte Ausgangsstoffe III sind Chlor- und Bromwasserstoffsäuren. Als Nitrosylhalogenide werden solche der Formel

$$O = N—X \qquad (V)$$

worin X ein Halogenatom, bevorzugt ein Chlor- oder Bromatom bedeutet, verwendet. Bevorzugte Alkalinitrite sind Natrium- und Kaliumnitrit. Bevorzugte Alkylnitrite sind solche der Formel

$$O = N—O—R^5 \qquad (VI)$$

worin $R^5$ einen Alkylrest mit 1 bis 6 Kohlenstoffatomen bedeutet, zweckmäßig Amylnitrit, Ethylnitrit, Neopentylnitrit. Verfahren a1) (Tetrafluorborwasserstoffsäure) wird zweckmäßig mit organischen Lösungsmitteln oder bevorzugt mit Gemischen von zusätzlichem Wasser und organischen Lösungsmitteln durchgeführt. Verfahren a2) kann mit organischen Lösungsmitteln und gegebenenfalls im Gemisch mit zusätzlichem Wasser, zweckmäßig aber in wäßrigem Medium durchgeführt werden. Außerdem entsteht bei Verfahren a) und b) Reaktionswasser. Bevorzugt verwendet man Alkalinitrite in Anwesenheit von Wasser oder wasserhaltigen organischen Lösungsmitteln. Alkylnitrite werden zweckmäßig in Gegenwart von organischen Lösungsmitteln und in Abwesenheit von Wasser eingesetzt.

Anstelle der Ausgangstoffe V kann man auch die Reaktionsgemische ihrer Herstellung, z. B. und NO und Halogen, insbesondere Brom, verwenden. Zweckmäßig setzt man das Stickoxid zu einer Lösung des Halogens in einem der nachgenannten Lösungsmittel, z. B. Methylenbromid, vorteilhaft in einem Verhältnis von 2,1 bis 3 Mol NO je Mol Halogen während 20 bis 60 Minuten bei — 15 bis — 5 °C zu und läßt das Gemisch noch 20 bis 30 Minuten bei — 15 bis — 10°C reagieren. Mit diesem Gemisch wird dann das erfindungsgemäße Verfahren durchgeführt.

Man kann die Ausgangsstoffe in stöchiometrischer Menge oder jeder der Komponenten jeweils im Überschuß umsetzen, vorzugsweise in einem Verhältnis von 1 bis 3, insbesondere von 1,05 bis 1,2 Mol Alkalinitrit oder Alkylnitrit, vorzugsweise 1 bis 3, insbesondere von 1,05 bis 1,2 Mol $HBF_4$ oder von 1 bis 3, insbesondere 1,05 bis 1,5 Mol Ausgangsstoff V je Mol Ausgangsstoff II. Man verwendet 1 bis 5, vorzugsweise 2 bis 4 Mol Ausgangstoff III je Mol Ausgangstoff II. Bevorzugte Ausgangsstoffe II, III, IV und V und dementsprechend bevorzugte Endstoffe I sind solche, in deren Formeln A ein Brom-, Chlor- oder Fluoratom oder eine Cyangruppe bezeichnet, $R^1$, $R^2$ und $R^3$ gleich oder verschiedenen sein können und jeweils einen unsubstituierten oder durch Alkoxy- und/oder Alkylthiogruppen mit zweckmäßig 1 bis 4 Kohlenstoffatomen, Halogenatome, bevorzugt Chlor- oder Fluoratome, Carbonamidogruppen, und/oder Carbalkoxygruppen mit zweckmäßig 2 bis 6 Kohlenstoffatomen, insbesondere eine dieser Gruppen oder Atome substituierten Alkylrest mit 1 bis 20, vorzugsweise 1 bis 8, insbesondere 1 bis 4 Kohlenstoffatomen oder einen Alkenylrest mit 2 bis 20, vorzugsweise 2 bis 8, insbesondere 2 bis 4 Kohlenstoffatomen, einen Cycloalkylrest mit 5 bis 8 Kohlenstoffatomen, einer Alkylaryl- oder Aralkylrest mit 7 bis 12 Kohlenstoffatomen oder einen Phenylrest bedeuten, $R^1$ und $R^2$ auch jeweils ein Wasserstoffatom bedeuten können, Y für einen Sauerstoffatom oder ein Schwefelatom steht, $R^4$ ein Wasserstoffatom oder den Rest $R^3Y$ bedeutet, worin $R^3$ und Y die vorgenannten Bedeutungen besitzen, und X ein Chloratom, Fluoratom oder Bromatom bezeichnet. Die vorgenannten Reste können noch durch unter den Reaktionsbedingungen inerte Gruppen oder Atome, z. B. Alkylgruppen, Alkoxi-, Alkylthiogruppen mit jeweils 1 bis 4 Kohlenstoffatomen, Chloratome, Bromatome substituiert sein.

Es kommen z. B. als Ausgangsstoffe II in Betrach : In 3- oder 5-Stellung einfach oder in 3- und 5-Stellung gleich oder unterschiedlich zweifach durch die Methyl-, Ethyl-, Propyl-, Isopropyl-, Butyl-, Isobutyl- sek.-Butyl-, Cyclohexyl-, Benzyl-, Phenyl-, Methoxymethyl-, Ethoxymethyl-, 2'-Methoxyethyl-, 2'-Ethoxyethylgruppe substituierte 2-Aminopyrazine ; unsubstituiertes 2-Aminopyrazin ; in 3-Stellung und 5-Stellung unsubstituierte, in 6-Stellung durch Methoxy-, Ethoxy-, Propoxy-, Isopropoxy-, Methylthio-, Ethylthio-, Propylthiogruppe substituierte 2-Aminopyrazine ; in 3,5,6-Stellung dreifach durch vorgenannte Gruppen in diesen Stellungen substituierte 2-Aminopyrazine.

Die Umsetzung a) bzw. b) wird bei einer Temperatur von — 50 bis + 50°C, im Falle des Verfahrens a) zweckmäßig von — 30 bis + 40, insbesondere von — 20 bis + 25 °C, im Falle des Verfahrens b) zweckmäßig von — 25 bis + 40, insbesondere von — 25 bis 0 °C, mit Unterdruck oder Überdruck oder drucklos, diskontinuierlich oder kontinuierlich durchgeführt. Man verwendet für Verfahren a) und b) Lösungsmittel. Wasser wird ganz oder teilweise in Gestalt von Lösungen der Säuren III und vorteilhaft in Gestalt von Lösungen des Alkalinitrits der Reaktion zugeführt. Als organische Lösungsmittel kommen im

allgemeinen je nach Verfahren a1), a2) oder Verfahren b) in Frage :

Halogenkohlenwasserstoffe (bevorzugt b), insbesondere Chlorkohlenwasserstoffe, z. B. Tetrachlorethylen, 1,1,2,2- oder 1,1,1,2-Tetrachlorethan, Amylchlorid, Dichlorpropan, Methylenchlorid, Dichlorbutan, Ethylchlorid, Isopropylbromid, n-Propylbromid, Butylbromid, Chloroform, Tetrachlorkohlenstoff, 1,1,1- oder 1,1,2-Trichlorethan, Trichlorethylen, 1,2-Dichlorethan, 1,1-Dichlorethan, n-Propylchlorid, n-Butylchlorid, 2-, 3- und iso-Butylchlorid, Chlorbenzol, Fluorbenzol, Brombenzol, o-, p- und m-Dichlorbenzol, o-, p-, m-Dibrombenzol, o-, m-, p-Chlortoluol, 1,2,4-Trichlorbenzol ; Ether (bevorzugt a1, a2), z. B. Ethylpropylether, n-Butylethylether, Di-n-butylether, Diisobutylether, Diisopropylether, Diethylether, Tetrahydrofuran, Dioxan ; Alkanole und Cycloalkanole (bevorzugt a1, a2) wie Ethanol, Methanol, n-Butanol, Isobutanol, tert.-Butanol, Glykol, sek.-Butanol, n-Propanol, Isopropanol, Cyclohexanol, 2-Ethylhexanol, Methylglykol, n-Hexanol, Isohexylalkohol, Isoheptylalkohol, n-Heptanol, Ethylbutanol, Nonylalkohol, Dodecylalkohol, Methylcyclohexanol, insbesondere solche mit 1 bis 4 Kohlenstoffatomen ; Ester (bevorzugt in Abwesenheit zusätzlicher Wassermengen), z. B. Essisgsäureethylester, Ameisensäuremethylester ; Sulfoxide wie Dimethylsulfoxid, Diethylsulfoxid, Dimethylsulfon, Diethylsulfon, Methylethylsulfon, Tetramethylensulfon ; Carbonsäuren (bevorzugt a1, a2) mit 1 bis 6 Kohlenstoffatomen, z. B. Essigsäure, Propionsäure, Ameisensäure und entsprechende Gemische von zueinander inerten Lösungsmitteln. Bevorzugt sind Diethylether, Essigsäure, Propionsäure, Ethanol, Butanol, Methylenchlorid, -bromid, Chlorbenzol. Zweckmäßig verwendet man das organische Lösungsmittel und/oder zusätzliches Wasser (nicht Reaktionswasser) in einer Menge von 50 bis 5 000 Gewichtsprozent, vorzugsweise von 100 bis 1 000 Gewichtsprozent, bezogen auf Ausgangsstoff II. Man kann einen Teil des Lösungsmittels oder die Gesamtmenge auch in Gestalt der entsprechenden Lösung der Ausgangsstoffe, z. B. der Tetrafluorborsäure, einsetzen. Ausgangsstoff III wird in Form einer 10- bis 80-, vorzugsweise 30- bis 70-gewichtsprozentigen Lösung verwendet.

Die Reaktion nach a) und b) kann wie folgt durchgeführt werden : Ein Gemisch von Ausgangsstoff II, a1) Alkylnitrit, Tetrafluorborsäure und organischem Lösungsmittel, a2) Ausgangsstoff III, Alkalinitrit und Wasser oder b), Ausgangsstoff V und organischem Lösungsmittel wird während einer Reaktionszeit von 0,2 bis 5 Stunden bei der Reaktionstemperatur gehalten. Dann wird aus dem Reaktionsgemisch der Endstoff in üblicher Weise, z. B. durch Destillation oder Extraktion der wäßrigen Phase und Destillation des Lösungsmittel, isoliert. Neben den Endstoffen I entsteht meist auch das entsprechende Pyrazinon als Nebenprodukt, das nach bekannten Verfahren, z. B. mit Phosphorhalogeniden (JACS, loc. cit.), in den Endstoff I umgewandelt werden kann. So können bis zu 90 % des Umsetzungsproduktes als Endstoff I weiterverarbeitet werden ; überraschend liefert das erfindungsgemäße Verfahren daher bis zu 90 % an Wertprodukt.

In einer bevorzugten Ausführungsform wird das 2-Aminopyrazin II in einer konzentrierten wäßrigen Halogenwasserstoffsäure oder einem die Halogenwasserstoffsäure bzw. die Tetrafluorborwasserstoffsäure enthaltenden organischen Lösungsmittel suspendiert bzw. gelöst, auf — 20 bis — 10 °C abgekühlt und anschließend unter weiterer Kühlung eine wäßrige Natriumnitritlösung bzw. ein Alkylnitrit langsam zugegeben. Dabei tritt Stickstoff-Entwicklung auf. Anschließend läßt man zur Nachreaktion die Temperatur auf Raumtemperatur ansteigen bzw. erwärmt gegebenenfalls noch auf 30 bis 50 °C. Das Reaktionsgemisch wird dann mit verdünnter Alkalilauge neutralisiert, wobei sich im allgemeinen 2 Phasen ausbilden. Zur Aufarbeitung extrahiert man die vorher abgetrennte wäßrige Phase mit einem geeigneten organischen Lösungsmittel, z. B. Methylenchlorid, und destilliert die vereinigten organischen Phasen gegebenenfalls nach vorheriger Trocknung zur Trennung des Endstoffs und des 2-Pyrazinon-nebenstoffs.

Höhere Ausbeuten an Halogenpyrazinen erhält man im allgemeinen bei Verfahren b).

In einer bevorzugten Ausführungsform arbeitet man mit Nitrosylbromid im Eintopfverfahren. Zunächst wird das in einem geeigneten Lösungsmittel, z. B. einem der vorgenannten, vorgelegte Brom durch Einleiten von NO bei + 10 °C bis — 40 °C, vorzugsweise — 10 °C bis — 20 °C, in NOBr überführt und anschließend das in einem vorgenannten Lösungsmittel gelöste 2-Aminopyrazin II bei der gleichen Temperatur zudosiert. Dabei wird im allgemeinen rasch $N_2$ gebildet.

Man kann den Ausgangsstoff II nach bekannten Verfahren, z. B. den in Recent Advances in Pyrazine Chemistry (loc. cit.) beschriebenen, herstellen. Zweckmäßig verwendet man die in der deutschen Patentanmeldung P 32 42 195.8 beschriebene Umsetzung von α-Iminodiacetonitrilen der Formel VII

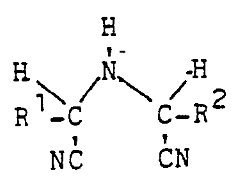

(VII)

1a) mit Halogenwasserstoffen der Formel III,

H—X (III)

oder

1b) mit Alkoholen und/oder Thioalkoholen der Formel VIII

$$HYR^3 \quad (VIII)$$

und Halogenwasserstoffen der Formel III oder

1c) mit Alkoholen und/oder Thioalkoholen der Formel VIII in Gegenwart von Alkali- und/oder Erdalkaliverbindungen

zu den 2-Aminopyrazinen II.

Die Reste $R^1$, $R^2$, $R^3$, X und Y haben die vorgenannten allgemeinen und bevorzugten Bedeutungen. Ausgangsstoffe II mit $R^4 = H$ werden nach Verfahren 1a oder 1b, mit $R^4 = R^3—Y—$ nach Verfahren 1c hergestellt.

Man kann die Ausgangsstoffe in stöchiometrischer Menge oder jede der Komponenten jeweils im Überschuß zur anderen umsetzen, vorzugsweise in einem Verhältnis im Falle des Verfahrens 1a) von 1,5 bis 6, insbesondere 2 bis 5 Mol Ausgangsstoff III je Mol Ausgangstoff VII, im Falle des Verfahrens 1b) von 1,5 bis 6, insbesondere 2 bis 5 Mol Ausgangsstoff III und/oder 0,1 bis 10, insbesondere 0,5 bis 3 Mol Ausgangsstoff VIII je Mol Ausgangsstoff VII, im Falle des Verfahrens 1c) von 5 bis 50, insbesondere 10 bis 25 Gew.% Stoff VII je Gewichtsmenge Stoff VIII.

Die Umsetzung (1a, 1b, 1c) wird vorteilhaft bei einer Temperatur von 0 bis 100 im Falle der Umsetzung 1a) zweckmäßig von 40 bis 80 °C, im Falle der Umsetzung b) zweckmäßig von 20 bis 80, bevorzugt von 30 bis 70 °C, im Falle der Umsetzung 1c) zweckmäßig von 0 bis 80, bevorzugt 20 bis 60 °C, mit Unterdruck oder Überdruck oder vorzugsweise drucklos, diskontinuierlich oder kontinuierlich durchgeführt. Die Reaktionszeit beträgt zweckmäßig 0,1 bis 200, vorzugsweise 3 bis 48 Stunden. Man verwendet gegebenenfalls unter den Reaktionsbedingungen inerte, organische Lösungsmittel. Als Lösungsmittel kommen z. B. in Frage : aromatische Kohlenwasserstoffe ; aliphatische oder cycloaliphatische Kohlenwasserstoffe ; Halogenkohlenwasserstoffe, insbesondere Chlorkohlenwasserstoffe ; Ether ; Mercaptane. Zweckmäßig verwendet man das Lösungsmittel in einer Menge von 40 bis 10 000 Gewichtsprozent, vorzugsweise von 50 bis 1 500 Gewichtsprozent, bezogen auf Ausgangsstoff VII. Man kann gegebenenfalls auch den Ausgangsstoff VIII selbst als Lösungsmittel nehmen.

Die Umsetzung 1c) wird in Gegenwart einer Hilfsbase, vorteilhaft in katalytischen Mengen, zweckmäßig in einer Menge von 0,1 bis 2, vorzugsweise 0,2 bis 0,6 Äquivalenten Hilfsbase, bezogen auf ein Mol Ausgangsstoff VII, durchgeführt. Die Hilfsbasen sind Erdalkaliverbindungen und insbesondere Alkaliverbindungen, vorteilhaft Alkoholate, Mercaptide, Hydroxide oder Cyanide, sowie entsprechende Gemische.

Man kann das 2-Aminopyrazin II aus dem Reaktionsgemisch seiner Herstellung isolieren, reinigen und dann für das erfindungsgemäße Verfahren einsetzen ; man kann aber auch das Reaktionsgemisch, gegebenenfalls nach Abtrennung der Lösungsmittel, einsetzen. Z. B. kann man das rohe 2-Aminopyrazin dieser Umsetzung, das noch einen Anteil bis zu 20 Gewichtsprozent 2-Halogenpyrazin, bezogen auf 2-Aminopyrazin, enthalten kann, direkt der erfindungsgemäßen Umsetzung unterwerfen. Solche 2-Halogenpyrazine wirken im allgemeinen lösend an das erfindungsgemäße Reaktionsgemisch.

In einer weiteren bevorzugten Ausführungsform wird das so erhaltene 2-Halogenpyrazin IV bzw. 2-Halogenpyrazin I in die 2-Cyanverbindung umgewandelt. Die allgemeinen und bevorzugten Bedeutungen der Reste $R^1$, $R^2$, $R^4$ und X sind die schon vorgenannten Bedeutungen der Reste bei den Ausgangsstoffen II. Der Stoff IV kann mit Kupfercyanid allein in stöchiometrischer Menge oder zweckmäßig im Überschuß, vorteilhaft in einer Menge von 1 bis 2, bevorzugt 1,05 bis 1,5 Mol Kupfercyanid je Mol Ausgangsstoff IV umgesetzt werden. Anstelle eines Anteils an Kupfercyanid können Alkali- und/oder Erdalkalicyanide, zweckmäßig Lithiumcyanid, Kalziumcyanid, Bariumcyanid, bevorzugt Natriumcyanid oder Kaliumcyanid, verwendet werden, zweckmäßig in Mengen von 1 bis 3 Mol, vorzugsweise 1,1 bis 1,5 Mol Alkali- und/oder Erdalkalicyanide je Mol Ausgangsstoff IV. Vorteilhaft sind Gemische von 10 bis 50, bevorzugt 15 bis 25 Mol.% Kupfercyanid, bezogen auf 100 Mol.% aller Cyanide. Die Umsetzung wird bei einer Temperatur von 80 bis 200 °C, vorzugsweise 120 bis 160 °C, drucklos, unter Überdruck oder Unterdruck, kontinuierlich oder diskontinuierlich durchgeführt. Man verwendet zweckmäßig unter den Reaktionsbedingungen inerte heterocyclische Lösungsmittel, zweckmäßig in einer Menge von 100 bis 5 000, bevorzugt 200 bis 1 000 Gewichtsprozent Lösungsmittel, bezogen auf Ausgangsstoff II. Als heterocyclische Lösungsmittel kommen in Betracht : Tetrahydrofuran, Dioxan, 1,3-Dioxolan, N-Methylpyrrolidon, Chinolin, 2,3-, 2,4-, 2,5-, 2,6-, 3,4-, 3,5-Dimethylpyridin, 2-, 3-, 4-Ethylpyridin, 2,4,6-Trimethylpyridin, 2-Methyl-5-ethylpyridin, 2-Propylpiperidin, Chinaldin, N-Propylpiperidin, N-Methylpyrrolidon, 2-Cyanpyrazine, bevorzugt $\gamma$-Picolin, Pyridin, N-Methylpyrrolidon.

Die Reaktion kann wie folgt durchgeführt werden : Ein Gemisch von Stoff IV, CuCN und heterocyclischem Lösungsmittel und gegebenenfalls einem Alkali- oder Erdalkalicyanid wird während 1 bis 12 Stunden bei der Reaktionstemperatur gehalten. Dann wird der Endstoff aus dem Gemisch in üblicher Weise, z. B. durch Vermischen des Reaktionsgemische mit Wasser und Lösungsmittel, z. B. Chloroform, Ansäuern, Filtration, Extraktion der wäßrigen Phase mit z. B. Chloroform und fraktionierte Destillation der vereinigten organischen Phasen, abgetrennt.

Die nach dem Verfahren der Erfindung erhältlichen 2-Halogen- und 2-Cyanpyrazine sind wertvolle Ausgangsstoffe für die Herstellung von Farbstoffen, Fungiziden, Bakteriziden, Textilhilfsmitteln und

Pharmazeutika. Sie sind Zwischenprodukte für die Folsäuresynthese. Bezüglich der Verwendung wird auf die vorgenannten Druckschriften und Ullmanns Encyklopädie der technischen Chemie, (4. Auflage), Band 9, Seiten 710 bis 711, verwiesen.

In einer bevorzugten Ausführungsform werden die symmetrischen und die früher nicht herstellbaren asymmetrischen Ausgangsstoffe VII nach der in der deutschen Patentanmeldung P 32 42 193.1 beschriebenen Arbeitsweise durch Umsetzung von Aldehydcyanhydrinen der Formel

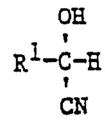 (IX)

mit Aminonitrilen der Formel

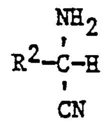 (X)

hergestellt. Im Falle von asymmetrischen und insbesondere von symmetrischen α-Iminodiacetonitrilen VII setzt man vorteilhaft in Gegenwart von niederen Alkanolen in einem Verhältnis von 5 bis 95 Gew.% Ausgangsstoff X, bezogen auf die Gewichtsmenge an niederem Alkanol, um. Die Herstellung von α-Iminodiacetonitrilen VII kann auch durch Umsetzung von Halogenacetonitrilen der Formel

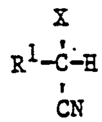 (XI)

mit Aminonitrilen X in Gegenwart einer Hilfsbase durchgeführt werden. Die vorgenannten allgemeinen und bevorzugten Bedeutungen der Reste $R^1$, $R^2$ und X haben auch für die Herstellung des Ausgangsstoffe VII ihre Gültigkeit. Vorzugsweise setzt man 1 bis 2, insbesondere 1 bis 1,2 Mol Ausgangsstoff IX oder 1 bis 1,5, insbesondere 1 bis 1,2 Mol Ausgangsstoff XI je Mol Ausgangsstoff X ein. Die Umsetzung wird zweckmäßig bei einer Temperatur von 0 bis 100, vorzugsweise 20 bis 60 °C, drucklos oder unter Druck, kontinuierlich oder diskontinuierlich, vorteilhaft in Gegenwart von 1 bis 2 Mol Gesamtwasser je Mol Ausgangsstoff IX durchgeführt. Als Hilfsbasen kommen zweckmäßig tertiäre Amine, vorteilhaft in einer Menge von 1 bis 2 Äquivalenten Amin, bezogen auf Stoff XI, in Frage. Auch organische inerte Lösungsmittel, z. B. Ether, zweckmäßig in einer Menge von 100 bis 10 000 Gew.%, bezogen auf Ausgangsstoff XI, können bei Verwendung des Stoffes XI verwendet werden.

Der so erhaltene Stoff VII kann aus dem Reaktionsgemisch in üblicher Weise isoliert und gereinigt werden, z. B. durch Destillation oder Extraktion ; er kann aber auch in Form des Reaktionsgemisches, gegebenenfalls nach z. B. destillativer Entfernung der Lösungsmittel, weiterverwendet werden. Die reinen oder rohen Stoffe VII werden dann in vorgenannter Weise zu den reinen oder rohen Ausgangsstoffen II und diese dann zu den Endstoffen I umgesetzt. Durch diese elegante Arbeitsweise in 3 Stufen werden zahlreiche Synthesewege auf den vorgenannten Industriegebieten, z. B. auch für Folsäureverbindungen, überraschend erschlossen bzw. wirtschaftlicher und einfacher.

Beispiel 1

3,5-Dimethyl-2-chlorpyrazin

12,3 g (100 mMol) 3,5-Dimethyl-2-aminopyrazin wurden in 40,55 g (400 mMol) 38 gew.%iger, wäßriger HCl in einem Rührgefäß suspendiert. Bei — 18 °C ließ man dann während 15 Minuten 20,7 g (120 mMol) wäßrige Natriumnitrit-Lösung (40 gew.%ig) zutropfen. Nach dem Anwärmen der Reaktionsmischung während 25 Minuten auf 22 °C wurde das Gemisch mit 61 g Natronlauge (20 gew.%ig) neutralisiert und nach Abtrennen der organischen Phase die wäßrige Phase mit Methylenchlorid extrahiert. Nach dem Einengen wurden die vereinigten organischen Phasen bei 20 mbar fraktioniert destilliert. Es wurde 6,87 g (48,2 % d. Th.) 2-Chlor-3,5-dimethylpyrazin ($Kp_{20}$ = 87 °C ; $n^{20}_D$ = 1,520 1) und aus dem Destillationsrückstand durch Umkristallisation aus Methanol 3,97 g (32 % d. Th.) 3,5-Dimethylpyrazinon-2 (Fp = 153 °C) erhalten, was einer Gesamtausbeute von 80,2 % der Theorie an Wertprodukt entspricht.

Beispiel 2

3,5-Dimethyl-2-fluor-6-methoxypyrazin

In einem Rührgefäß wurden 15,32 g (100 mMol) 3,5-Dimethyl-6-methoxy-2-aminopyrazin in 34,3 g (210 mMol) Tetrafluorborvasserstoffsäure (54 gew.%ig in Diethylether) und 20 g Diethylether gelöst. Bei — 20 °C ließ man eine Lösung von 17,25 g (100 mMol) $NaNO_2$ (40 gew.%ig in $H_2O$) langsam während 25 Minuten unter gutem Rühren zutropfen. Dann neutralisierte man das Gemisch mit Sodalösung, trennte von der waßrigen Phase ab und destillierte die organische Phase nach Verdampfen des Lösungsmittels fraktioniert. Es wurden 9,6 g (61,5 % d. Th.) 3,5-Dimethyl-2-fluor-6-methoxy-pyrazin ($Kp_{40\ mbar}$ = 83 °C ; Fp = 24 °C) erhalten. Aus der wäßrigen Phase erhielt man durch Extraktion mit Methylenchlorid noch zusätzlich 3,31 g (21,6 %) 3,5-Dimethyl-6-methoxypyrazinon-2 (Fp = 158 °C), so daß sich eine Gesamtausbeute von 83,1 % an Wertprodukt ergab.

Beispiel 3

3,5-Dimethyl-2-fluor-pyrazin

Führte man die in Beispiel 2 beschriebene Reaktion unter den gleichen Reaktionsbedingungen mit 3,5-Dimethyl-2-amino-pyrazin durch, so erhielt man 57 % 3,5-Dimethyl-2-fluor-pyrazin ($Kp_{100\ mbar}$ = 78 °C ; $n^{20}_D$ = 1,473 3) neben 24,8 % 3,5-Dimethylpyrazinon-2.

Beispiel 4

3,5-Dimethyl-2-chlor-pyrazin

98,3 g (1,5 Mol) Nitrosylchlorid wurden in 200 g Methylenchlorid bei — 10 °C bis — 20 °C gelöst. Dann ließ man bei — 20 °C unter gutem Rühren 123,2 g (1 Mol) 3,5-Dimethyl-2-aminopyrazin, das vorher in 600 g $CH_2Cl_2$ gelöst wurde, während 40 Minuten zulaufen. Anschließend wurden bei 20 mbar die gelösten Restgase abgezogen, die Reaktionslösung mit 175,6 g NaOH auf einen pH-Wert von 8,5 eingestellt und die organische Phase abgetrennt. Nach Extraktion der wäßrigen Phase mit Methylenchlorid und Verdampfen des Lösungsmittels wurden die vereinigten organischen Phasen fraktioniert destilliert. Ausbeute : 105,5 g (= 74 % d. Th.) 3,5-Dimethyl-2-chlor-pyrazin neben 15 g (12,1 % d. Th.) 3,5-Dimethylpyrazinon-2.

Beispiel 5

3,5-Dimethyl-2-chlor-6-methoxypyrazin

9,8 g (150 mMol) Nitrosylchlorid wurden in 100 g $CH_2Cl_2$ bei tiefer Temperatur gelöst und bei — 20 °C bis — 15 °C mit 15,35 g (100 mMol) 3,5-Dimethyl-2-amino-6-methoxypyrazin, wie im Beispiel 4 ausgeführt, umgesetzt.

Nach der Aufarbeitung (Extraktion mit Methylenchlorid) wurden 10,96 g (70 % d. Th.) 3,5-Dimethyl-2-chlor-6-methoxypyrazin (Fp = 48 °C) neben 1,46 g (9,5 % d. Th.) 3,5-Dimethyl-6-methoxypyrazinon-2 (Fp = 158 °C) isoliert.

Beispiel 6

3-Ethyl-5-methyl-2-chlorpyrazin

Man setzte 14,5 g (100 mMol) 3-Ethyl-5-methyl-2-aminopyrazin, bei — 20 °C bis — 18 °C mit 9,8 NOCl in 100 g $CH_2Cl_2$, wie im Beispiel 4 ausgeführt, um ; nach einer Nachreaktionszeit von 50 Minuten bei 0 °C neutralisierte man die Reaktionslösung, extrahierte mit $CH_2Cl_2$ und destillierte das Gemisch. Ausbeute : 10,18 g (65 % d. Th.) 3-Ethyl-5-methyl-2-chlorpyrazin ($Kp_{20\ mbar}$ = 85 °C) neben 1,38 g (10 % d. Th.) 2-Ethyl-5-methyl-pyrazinon-2 (Fp = 96 °C), das aus der wäßrigen Phase erhalten wurde.

Beispiel 7

3-Isobutyl-2-chlor-pyrazin

Analog Beispiel 4 ließ man 15,4 g (100 mMol) 3-Isobutyl-2-amino-pyrazin mit 9,8 g NOCl in 100 g Methylenchlorid bei — 15 °C bis — 20 °C reagieren. Nach 30 Minuten bei 0 °C wurde die Reaktionslösung aufgearbeitet.

Ausbeute : 11,26 g (66 % d. Th.) 3-Isobutyl-2-chlorpyrazin ($Kp_{10\ mbar}$ = 78 °C).

Beispiel 8

3,5-Dimethyl-2-brom-pyrazin

96 g (0,6 Mol) Brom wurden in 100 g Methylenbromid gelöst. Bei — 19 °C wurden im Verlauf von 1,5 Stunden 36 g (1,2 Mol) Stickstoffmonoxid eingeleitet. Die Reaktion war schwach exotherm. Nach Beendigung der Einleitung wurde das Gemisch noch 1 Stunde bei — 10 °C bis — 20 °C nachgerührt. In die Nitrosylbromidlösung ließ man dann 123 g (1 Mol) 3,5-Dimethyl-2-amino-pyrazin, das in 750 g Methylenbromid gelöst wurde, zutropfen. Die Zulaufzeit betrug 1,5 Stunden. Durch Kühlung wurde die Temperatur auf 5 °C gehalten. Nun wurde das Gemisch 30 Minuten bei 25 °C nachgerührt. Dann wurden 50 ml $H_2O$ zugesetzt. Bei 15 °C stellte man das Gemisch mit 97,6 g (0,61 Mol) Natronlauge (20 Gew.%ig) auf pH 8,5. Nach Abtrennung der organischen Phase und Extraktion der wäßrigen Phase mit Methylenbromid wurden die vereinigten organischen Phasen eingeent und fraktioniert destilliert.

Die Ausbeute an 3,5-Dimethyl-2-brom-pyrazin betrug 85 % d. Th. (Fp 7 °C ; $n^{20}_D$ = 1,559 0).

Beispiel 9

3,5-Dimethyl-6-methoxy-2-brompyrazin

In 25 g Methylenbromid löste man unter guter Kühlung (— 15 °C bis — 20 °C) 9,6 g (60 mMol) Brom und 3,6 g (120 mMol) NO. Dann ließ man unter weiterer Kühlung und gutem Rühren im Laufe einer Stunde 17,3 g (100 mMol) 3,5-Dimethyl-6-methoxy-2-amino-pyrazin, das in 120 g Methylenbromid gelöst war, bei — 18 °C gleichmäßig zulaufen. Nach 1 Stunde Nachreaktionszeit bei 20 °C gab man bei derselben Temperatur 50 ml Wasser zu und neutralisierte mit 28,1 g Natronlauge (20 gew.%ig) auf pH 7,5. Nach Extraktion der wäßrigen Phase mit Methylenbromid, Verdampfen des Lösungsmittels und anschließender fraktionierter Destillation erhielt man 17,13 g 3,5-Dimethyl-6-methoxy-2-brompyrazin bei $Kp_{10\ mbar}$ 97 °C. Ausbeute : 78,9 % d. Th.

Beispiel 10

2-Brom-6-methoxypyrazin

Man legte 28,8 g (180 mMol) Brom in 75 g Chlorbenzol vor, leitete bei — 10 °C 10,8 g (360 mMol) NO ein und ließ anschließend unter Rühren bei — 15 °C bis — 10 °C 37,6 g (300 mMol) 2-Amino-6-methoxypyrazin, das in 300 g Chlorbenzol gelöst war, während 90 Minuten zutropfen. Nach 30 Minuten Nachreaktionszeit von — 10 bis + 22 °C wurde das Gemisch nach Zusatz von 20 ml $H_2O$ mit verdünnter Natronlauge neutralisiert, die organische Phase und die Methylenchloridextrakte der Wasserphase wurden dann destillativ aufgearbeitet. Man erhielt 45,3 g (74 % Ausbeute d. Th.) 2-Brom-6-methoxy-pyrazin ($Kp_{14\ mbar}$ 77 °C).

Beispiel 11

3-Isopropyl-5-methyl-2-brompyrazin

9,6 g (60 mMol) Brom in 25 g Methylenbromid wurden, wie in Beispiel 8 beschrieben, mit 120 mMol NO bei — 19 °C bis — 15 °C zu Nitrosylbromid umgesetzt. In das Gemisch ließ man bei — 20 °C bis — 13 °C eine Lösung von 15,5 g (100 mMol) 3-Isopropyl-5-methyl-2-aminopyrazin (97,5 gew.%ig) in 100 g Methylenbromid während 1,5 Stunden langsam zutropfen. Nach der Aufarbeitung analog Beispeil 8 wurden nach der Destillation 16,9 g (78,6 % d. Th.) 3-Isoproprył-5-methyl-2-brompyrazin erhalten ($Kp_{1,5\ mbar}$ = 61 °C ; Fp = 10 °C ; $n^{20}_D$ = 1,536 4).

Beispiel 12

3-n-Butyl-2-brompyrazin

Analog Beispiel 11 bei gleichen Molverhältnissen der Komponenten wurden 900 mMol NOBr (Methylenbromid) mit einer Lösung von 116,1 g (750 mMol) 3-n-Butyl-2-aminopyrazin (97,6 %ig) in 600 g Methylenbromid umgesetzt. Man erhielt 126,5 g 3-n-Butyl-2-brompyrazin vom $Kp_{0,5\ mbar}$ = 100 °C (78,4 % d. Th.).

Beispiel 13

3-Isobutyl-2-brompyrazin

Man führte die in Beispiel 11 beschriebene Umsetzung im entsprechenden stöchiometrischen Verhältnis mit 38.4 g (250 mMol) 3-Isobutyl-2-aminopyrazin (97,5 %ig) durch. Es wurden nach der Aufarbeitung 41,50 g (77,2 % Ausbeute d. Th.) 3-Isobutyl-2-brompyrazin ($Kp_{0.2\ mbar}$ = 56 °C ; $n^{20}_D$ = 1,537 4) erhalten.

Beispiel 14

Bei der Umsetzung von 9,25 g (50 mMol) 3-Methoxymethyl-5-methyl-6-methoxy-2-aminopyrazin mit Nitrosylbromid aus 4,8 g Brom und 1,8 g NO in insgesamt 60 g Methylenbromid analog Beispiel 11 gewinnt man 8,7 g (= 70,4 % d. Th. ($Kp_{1\ mbar}$ = 82 °C ; Fp = 41-43 °C) 3-Methoxymethyl-5-methyl-6-methoxy-2-brompyrazin.

Beispiel 15

2-Brompyrazin

Bei der Umsetzung von 9,5 g (100 mMol) 2-Aminopyrazin mit Nitrosylbromid aus 16 g Brom und 6 g NO in insgesamt 280 g Methylenbromid analog Beispiel 11 erhielt man 10,3 g (65 % d. Th.) 2-Brompyrazin ($Kp_{16}$ 58-59 °C).

Beispiel 16

3,5-Dimethyl-2-cyanopyrazin

37,4 g (200 mMol) 2-Brom-3,5-dimethylpyrazin, 21,5 g (240 mMol) CuCN und 160 ml γ-Picolin wurden unter Rühren 7 1/2 Stunden auf Rückflußtemperatur (Ölbad 92 bis 150 °C) erhitzt.

Das auf 85 °C abgekühlte Gemisch wurde auf ein Gemisch von 500 g Eis und 300 g $CHCl_3$ gegeben, mit 300 g 38 gew.%iger, wäßriger HCl angesäuert, filtriert und mit 3 × 100 g $CHCl_3$ die abgetrennte wäßrige Phase ausgeschüttelt. Nach Vereinigung der organischen Phasen und Verdampfen des Lösungsmittels wurde das Gemisch fraktioniert destilliert. Man erhielt 25,18 g 3,5-Dimethyl-2-cyanopyrazin (94,6 % der Th.) vom Kp = 109 °C (21 mbar) und Fp = 36 bis 38 °C.

Beispiel 17

3,5-Dimethyl-2-cyanopyrazin

Analog Beispiel 16, jedoch anstelle des CuCN mit 21,5 g einer Mischung von 83 Gew.% KCN + 17 Gew.% CuCN, erhielt man eine Ausbeute von 85,1 % d. Theorie an 3,5-Dimethyl-2-cyanopyrazin vom Kp = 109 °C (2,1 mbar).

Beispiel 18

3,5-Dimethyl-6-methoxy-2-cyanopyrazin

Aus 3,5-Dimethyl-6-methoxy-2-brom-pyrazin wurde analog Beispiel 16 das 3,5-Dimethyl-6-methoxy-2-cyanopyrazin ($Kp_{1\ mbar}$ = 72 °C ; Fp = 63 °C) in einer Ausbeute von 70 % der Theorie hergestellt.

Beispiel 19

3,5-Dimethyl-2-brom-pyrazin

Analog Beispiel 8 wurde die Umsetzung anstelle von 850 g Methylenbromid mit 1 000 g Chlorbenzol durchgeführt ; man erhielt 3,5-Dimethyl-2-brom-pyrazin vom Fp. 7 °C in einer Ausbeute von 82 % der Theorie.

**Patentanspruch**

Verfahren zur Herstellung von 2-Halogen- oder 2-Cyanopyrazinen der Formel

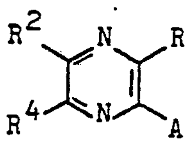

(I)

worin A ein Halogenatom oder eine Cyangruppe bedeutet, $R^1$ und $R^2$ gleich oder verschieden sein können und jeweils für ein Wasserstoffatom, einen aliphatischen, cycloaliphatischen, araliphatischen oder aromatischen Rest stehen, $R^4$ ein Wasserstoffatom oder den Rest $R^3Y$ bedeutet, worin $R^3$ für einen aliphatischen, cycloaliphatischen, araliphatischen oder aromatischen Rest steht und Y für ein Sauerstoffatom oder ein Schwefelatom steht, durch Umsetzung von 2-substituierten Pyrazinen mit Halogenverbindungen zu 2-Halogenpyrazinen und gewünschtenfalls Umsetzung von 2-Halogenpyrazinen mit Kupfercyanid in Gegenwart von unter den Reaktionsbedingungen inerten heterocyclischen Lösungsmitteln, dadurch gekennzeichnet, daß man 2-Aminopyrazine der Formel

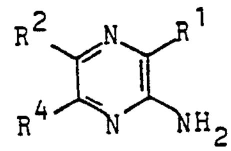

(II)

worin $R^1$, $R^2$ und $R^4$ die vorgennante Bedeutung besitzen, bei einer Temperatur von —50 °C bis + 50 °C,

a) mit Alkalinitriten oder Alkylnitriten in Gegenwart von Wasser und/oder organischen Lösungsmitteln a1) mit Tetrafluorborsäure oder a2) Halogenwasserstoffsäuren der Formel

H—X (III),

worin X ein Halogenatom bedeutet, in Form einer 10 bis 80 gewichtsprozentigen Lösung und mit Ausgangsstoff III in einer Menge von 1 bis 5 Mol Ausgangsstoff III je Mol Ausgangsstoff II, oder

b) mit Nitrosylhalogeniden in Gegenwart von unter den Reaktionsbedingungen inerten organischen Lösungsmitteln umsetzt und gewünschtenfalls die so erhaltenen 2-Halogenpyrazine der Formel

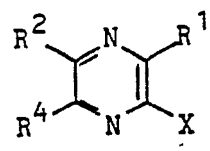

(IV)

worin $R^1$, $R^2$, $R^4$ und X die vorgenannte Bedeutung besitzen, mit Kupfercyanid und gegebenenfalls Alkali- und/oder Erdalkalicyaniden bei einer Temperatur von 80 bis 200 °C umsetzt.

**Claim**

A process for the preparation of a 2-halo- or a 2-cyanopyrazine of the formula

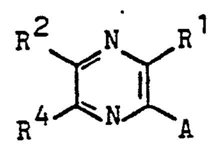

(I)

where A is halogen or cyano, $R^1$ and $R^2$ can be identical or different and are each hydrogen, an aliphatic, cycloaliphatic, araliphatic or aromatic radical, and $R^4$ is hydrogen or a radical $R^3Y$, where $R^3$ is an aliphatic, cycloaliphatic, araliphatic or aromatic radical, and Y is oxygen or sulfur, by reacting a 2-substituted pyrazine with a halogen compound to give a 2-halopyrazine and, if desired, reacting this product with copper cyanide in the presence of a heterocyclic solvent which is inert under the reaction conditions, wherein a 2-aminopyrazine of the formula

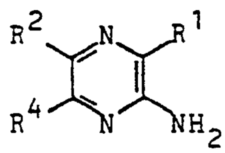

(II)

where $R^1$, $R^2$ and $R^4$ have the above meanings, is reacted at from —50 to + 50 °C

a) with an alkali metal nitrite or an alkyl nitrite in the presence of water and/or an organic solvent and a1) with tetrafluoboric acid or a2) a hydrohalic acid of the formula

H—X (III),

where X is halogen, in the form of a 10-80 per cent strength by weight solution, using from 1 to 5 moles of starting material III per mole of starting material II, or

b) with a nitrosyl halide in the presence of an organic solvent which is inert under the reaction

conditions, and, if desired, the resulting 2-halopyrazine of the formula

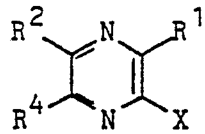

(IV)

where $R^1$, $R^2$, $R^4$ and X have the above meanings, is reacted with copper cyanide and, if desired, an alkali metal cyanide and/or an alkaline earth metal cyanide at from 80° to 200 °C.

**Revendication**

Procédé de préparation de 2-halogéno- ou 2-cyanopyrazines de formule

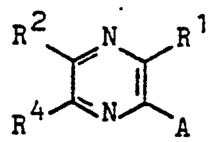

(I)

dans laquelle A représente un atome d'halogène ou un groupement cyano, $R^1$ et $R^2$ peuvent être identiques ou dissemblables et sont mis chacun pour un atome d'hydrogène, un radical aliphatique, cycloaliphatique, araliphatique ou aromatique, $R^4$ représente un atome d'hydrogène ou le radical $R^3Y$, $R^3$ étant mis pour un radical aliphatique, cycloaliphatique, araliphatique ou aromatique et Y pour un atome d'oxygène ou un atome de soufre, par la mise en réaction de pyrazines 2-substituées avec des composés halogénés pour l'obtention de 2-halogénopyrazines et, si on le désire, par réaction de 2-halogénopyrazines avec le cyanure de cuivre en présence de solvants hétérocycliques inertes dans les conditions de la réaction, caractérisé en ce qu'on fait réagir des 2-aminopyrazines de formule

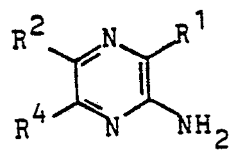

(II)

dans laquelle $R^1$, $R^2$ et $R^4$ ont les significations données ci-dessus, à une température de — 50 à + 50 °C,

a) avec des nitrites alcalins ou des nitrites d'alkyle en présence d'eau et/ou de solvants organiques et avec a1) de l'acide tétrafluoroborique ou a2) des hydracides halogénés de formule

$$H{-}X \qquad (III),$$

dans laquelle X représente un atome d'halogène, sous la forme d'une solution à 10-80 % en poids et, avec la substance de départ III, dans une proportion de 1 à 5 mol de substance de départ III par mol de substance de départ II ou

b) avec des halogénures de nitrosyle en présence de solvants organiques inertes dans les conditions de la réaction et, si on le désire, on fait réagir les 2-halogénopyrazines ainsi obtenues, de formule

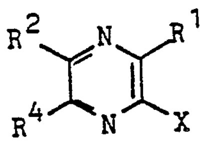

(IV)

dans laquelle $R^1$, $R^2$, $R^4$ et X ont les significations données ci-dessus, avec le cyanure de cuivre et, le cas échéant, des cyanures alcalins et/ou alcalinoterreux à une température de 80 à 200 °C.